# EUROPEAN PATENT APPLICATION

(11) **EP 1 891 926 A1**
(43) Date of publication of application: **27.02.2008**
(21) Application number: 06017356.4
(22) Date of filing: 21.08.2006
(51) Int. Cl.: A61K 8/34, A61K 8/36, A61K 8/60, A61Q 19/10

(54) **Body and hair cleansing formulations**

(71) Applicant: ACO Hud Nordic AB, 194 26 Upplands Väsby (SE)
(72) Inventor: Akerström, Ulf, S-117 62 Stockholm (SE); Buraczewska, Izabela, S-111 23 Stockholm (SE); Lodén, Marie, S-17077 Solnä (SE); Wärnheim, Torbjörn, S-16342 Spänga (SE)
(74) Representative: Holmberg, Nils Anders Patrik

(57) **Abstract**

The present invention generally relates to body and hair cleansing products especially intended for infants and kids, only containing constituents conventionally used as foodstuff additives.

## Description

### Field of the invention

The present invention generally relates to body and hair cleansing products, and particularly such products only containing constituents conventionally used as foodstuff additives. The products are especially intended for infants and children.

### Background art

Formulations for personal care are conventionally known and have been used for a very long time. Products such as shampoo and liquid soap, are well-established products belonging to this category. Generally, such products are formulated so as to have a smell which is attractive to the consumer. The products can for example be scented with apple, mango, raspberry, coconut and so on, or with various "parfum" type of fragrances. Such scents may however also give the impression, especially to kids, that the products are edible.

Moreover, very young kids tend to discover things by trying them in their mouth, including products such as shampoos and soaps, probably without even considering any edibility thereof.

### Summary of invention

The present inventors have found that shampoos and liquid soaps may be formulated entirely from foodstuff additives. By using only foodstuff additives, the products are believed to be less toxic and better tolerated by the human organism when mistakenly ingested. Also, as to toxicity, advantage is thereby taken of the fact that the constituents already have been tested and approved for use in food.

The formulation of the present invention is especially intended for use in products for infants and kids, but may of course also be used by adults.

Accordingly, in a first aspect the present invention relates to a cleansing formulation for personal use, containing only substances approved for use as foodstuff additives, said substances comprising, in addition to water, 10-50 % by weight of glycerol, and 5-25 % by weight of one or more surfactants selected from soaps and polysorbate 20.

In one embodiment, the formulation is formulated as a shampoo, preferably containing sorbitol.

In another embodiment, the formulation is formulated as a liquid soap.

In a preferred embodiment, the formulation is preservative free and contains at least 30 % by weight of glycerol.

Further embodiments and advantages will become apparent from the detailed description and appended claims.

### Detailed description

The use of additives in food items is generally strictly regulated by national and/or international authorities.

In order for an additive to be allowed for use in food, it will generally have to be approved by such authorities. In order for a substance to be allowed for use as a foodstuff additive within e.g. the E.C., it must have been approved for such use by the European Food Safety Authority (EFSA). There is also currently a cooperation on an international level, wherein the Joint FAO/WHO Expert Committee on Food Additives (JECFA) has an important role. If for example the JECFA has found an additive not acceptable, it will be very unlikely for such additive to be accepted within the E.C.

The substances used according to the present invention are substances approved by the EFSA, which substances appear on the so-called E list.

The purity of the substances used in the formulation of the inventions is not critical for proper function thereof. Thus, substances of both food grade and cosmetic grade can be used in the inventive formulations. However, while substances of food grade very well can be employed in the formulation of the invention, governmental regulations will typically prescribe that cosmetic grade must be used in cosmetic products, such as the products of the present invention. In some instances, cosmetic grade implies a higher purity than corresponding food grade substance.

### Ingredients

In its most generic form the formulation of the invention consists of 10-50 % by weight of glycerol, 5-25 % by weight of one or more surfactants selected from soaps and polysorbate 20, and water up to 100 %.

### Surfactant

The surfactant can be any combination of one or more soaps and optionally polysorbate 20 (E432).

The soap used can be any soap appearing on the E list. For the purpose of the E list, a soap is defined as being a salt of a free fatty acid. Examples of such soaps are potassium myristate, sodium laurate, potassium cocoate, and sodium oleate. An example of a preferred soap is potassium cocoate, which is a mixture of C₁₂-C₁₆ soaps, and which has good foaming properties.

Polysorbate can be used in the formulation for imparting an oily feeling. When used in combination with a soap, polysorbate 20 will also act as a solubilizer for the soap.

When the formulation is formulated as a shampoo it is preferred that sorbitol is included for imparting foaming, and hair improving properties, such as better combing properties. Sorbitol is preferably included in an amount of 0.25-2 % by weight.

### Glycerol

When used together with surfactants and foaming agents, glycerol i.a. improves the foaming properties of the resulting composition.

It has been found that by using high quantities glycerol the product will be self-preserved. In higher quantities it has an antimicrobial activity, since is lowers the water activity in the formulation, so that the there is less water available to micro-organisms to grow in. 40 % by weight of glycerol in the formulation is presently considered to be a suitable amount for achieving the desired antimicrobial effect. A lower amount of glycerol, such as for example about 30 % by weight, may however be sufficient to establish the effect. Such amount may be established by the skilled person using routine experimentation.

Accordingly, in one embodiment the inventive formulation is self-preserved by means of presence of a sufficient amount of glycerol.

### Stability of the inventive composition

The present inventors have found that as the glycerol content is increased, and, consequently, the water content decreased, the solubility of the other ingredients will become smaller. Accordingly, generally, when using a high glycerol content in the inventive formulation, such as required for self-preservation, only a small amount of the soaps can be stably dissolved in a formulation. If a larger amount of the soaps are employed in the formulation, the soaps will start to separate over time, due to crystallization thereof. This process may however be relatively slow, and may proceed for weeks or months.

In order to improve the stability of the formulation, polysorbate 20 could be used as the sole surfactant, since polysorbate 20 can be stably dissolved at high concentrations of glycerol. However, in order to obtain proper foaming properties, the addition of a soap will generally also be required, such as for example potassium cocoate. As already mentioned above, the polysorbate 20 will enhance the solubility of the soap. Thus, in order to obtain a balance of foaming properties and stability of the formulations of the present invention, it is generally preferred to include polysorbate 20 as the main surfactant. Preferably polysorbate 20 is included in a ratio of the amount of polysorbate 20 to that of the soaps of 1:1 to 10:1.

However, the solubilization action of polysorbate 20 has been found not to be sufficient for establishing the desired long-term stability of the formulation when high concentrations of glycerol is used, such as required for self-preservation.

It has surprisingly been found that the problem of crystallization can be solved by means of adding sodium oleate. The addition of sodium oleate will then further improve the solubility of potassium cocoate, and thus allowing for increased amounts of potassium cocoate to be dissolved in the formulation. Sodium oleate is typically used in an amount sufficient to produce the desired long term stability. The amount required will normally be within the range of 10 to 70 % of the total amount of soaps used, and can be established by the skilled person by routine experimentation.

### Viscosity

The formulation of the present invention is entirely liquid in itself, i.e. of a low viscosity. Therefore, in order to be of practical use, the formulation should be brought into a more convenient viscosity. Also, the formulation in itself is difficult to foam properly. These drawbacks can be readily overcome by means of using a foaming dispenser, which brings the formulation into a foam when dispensed. Such dispensers are commercially available. Accordingly, the soap or shampoo formulation can be contained in a suitable container, which container is provided with a foaming dispenser, such as foaming pump. Suitable foaming pumps, also referred to as pump foamers or foam pumps, are e.g. commercially available from Airspray N.V. (Alkmaar, The Netherlands).

The presence of glycerol in the range of the invention makes it difficult to increase the viscosity of the formulation. Thus, the presence of glycerol, especially in the high content required for self-preservation, will for example render the use of presently known thickening agents approved for use as food additives inefficient for the purpose of increasing the viscosity of the formulation.

With a suitable thickener for including in the formulation of the invention available, once approved for use as a food additive, that is, the present inventors believe the current foaming pump requirement could be dispensed with.

### pH of the compositions

When an amount of glycerol less than that required for self-preserving is employed, in order to prevent any possible fungi growth within the composition during long term storage thereof, it may be desirable to include a buffer in the composition for obtaining a higher pH value, at which pH value the growth of unwanted fungi will be prevented. Examples of suitable buffer systems are sodium carbonate/sodium bicarbonate and glycine/sodium hydroxide. It has also been found that even when glycerol is included in the composition in an amount effective for self-preservation of the composition, buffering of the system may further improve the stability of the system. Accordingly, in a preferred embodiment of the invention a buffer system is included in the composition. The desired pH value at which to buffer the system for obtaining the desired improved stability, will primarily be dependent on the soap(s) used. For example, when sodium oleate and potassium cocoate are used as the soaps, a pH of about 10 of the composition is desirable. When used, the buffer system will be included in the inventive composition in an amount effective for buffering the composition at the desired pH value.

### Preservatives

In an alternative embodiment the formulation of the invention could also contain preservatives. Thereby, the high glycerol content used for self-preservation will not be necessary, and the content of glycerol can consequently be reduced. Also, the above solubility problem associated with high glycerol can thereby be reduced. Suitable preservatives are benzoic acid, sorbic acid, and derivatives thereof, such as the sodium and calcium salts. However, from an allergenic view point the use of preservatives is not preferred, since preservatives are believed to be allergenic. Especially for the purpose of products intended for infants and kids, preservatives should be avoided.

The formulations can be prepared by merely mixing the ingredients together in any order of addition, especially since all the ingredients are water soluble. Powdery components may be heated in (part of) the water in order to enhance the solution process thereof. Accordingly, powdery sodium oleate may conveniently be heated in water to about 60°C.

The below examples are provided for illustrative purposes only, without limiting the invention thereto.

### Examples

### Example 1 - Preservative free liquid soap formulation

40 % by weight of glycerol;
13 % by weight of polysorbate 20;
2.0 % by weight of potassium cocoate;
2.0 % by weight of sodium oleate;
buffer system (sodium carbonate/sodium bicarbonate), q.s. for obtaining a pH value of about 10 of the resulting composition; water to 100 %.

The above components were mixed together, heating the powdery sodium oleate in part of the water as described above before admixing to the other components.

The soap formulation exhibited long-term stability, and foamed well when dispensed from a foaming pump.

### Example 2 - Preservative free shampoo formulation

40 % by weight of glycerol;
13 % by weight of polysorbate 20;
2.0 % by weight of potassium cocoate;
2.0 % by weight of sodium oleate;
0.5 % by weight of sorbitol;
q.s. of buffer system (sodium carbonate/sodium bicarbonate) for obtaining a pH value of about 10 of the resulting composition;
water to 100 %.

The above components were mixed together, heating the powdery sodium oleate in part of the water as described above before admixing to the other components.

The shampoo formulation exhibited long-term stability, and foamed well when dispensed from a foaming pump.

## Claims

1. Cleansing formulation for personal use, **characterised in** containing only substances approved for use as foodstuff additives, said substances comprising, in addition to water, 10-50 % by weight of glycerol, and 5-25 % by weight of one or more surfactants selected from soaps and polysorbate 20.

2. The cleansing formulation of claim 1, containing polysorbate 20 and one or more soaps.

3. The cleansing formulation of claim 1 or 2, containing potassium cocoate in an amount of 1-10, more preferably 1-5 % by weight.

4. The cleansing formulation of any of the previous claims, containing sodium oleate in an amount of 1-10, more preferably 1-5 % by weight.

5. The cleansing formulation of any of the previous claims, containing polysorbate 20 in an amount of 3-23, more preferably 8-18 % by weight.

6. Preservative free cleansing formulation of any of the previous claims, wherein glycerol is contained in an amount effective for preserving the formulation.

7. The cleansing formulation of any of claims 1-5, further containing benzoic acid, sorbic acid, or a derivative thereof, approved for use as a foodstuff additive.

8. The cleansing formulation of claim 7, further containing a thickener.

9. The cleansing formulation of any of the previous claims formulated as a shampoo product, preferably additionally containing sorbitol.

10. The cleansing formulation of any of the claims 1-8, formulated as a liquid soap product.

11. The cleansing formulation of any of the previous claims, additionally containing a buffer.
